# EUROPEAN PATENT SPECIFICATION

(11) **EP 3 814 138 B1**
(45) Date of publication and mention of the grant of the patent: **04.11.2026**
(21) Application number: 19827312.0
(22) Date of filing: 10.06.2019
(51) Int. Cl.: B32B 5/02, B32B 7/08, B32B 7/12, B32B 25/04, B32B 25/10, B32B 25/16, A61F 13/49

(54) **POLYDIMENSIONAL STRETCHABLE LAMINATES**
MEHRDIMENSIONALE DEHNBARE LAMINATE
STRATIFIÉS ÉTIRABLES POLYDIMENSIONNELS

(30) Priority: 29.06.2018 US 201862691738 P
(43) Date of publication of application: 05.05.2021
(60) Divisional application: 26206942.0
(73) Proprietor: Berry Global, Inc., Evansville, Indiana 47710 (US)
(72) Inventor: MUSLET, Iyad, West Chester, Ohio 45069 (US)
(74) Representative: Grünecker Patent- und Rechtsanwälte PartG mbB
(86) International application number: PCT/US2019/036257
(87) International publication number: WO 2020/005515

(56) References cited:
- EP-A1- 3 316 834
- EP-A2- 3 152 047
- WO-A2-2008/005822
- US-A- 5 422 172
- US-A- 5 695 868
- US-A1- 2007 237 924
- US-A1- 2010 168 704
- US-A1- 2017 000 660

## Description

### RELATED APPLICATIONS

This application claims the benefit of priority of U.S. Provisional Patent Application No. 62/691,738, filed June 29, 2018.

### FIELD OF THE INVENTION

The present invention relates to laminates which are recoverably stretchable in multiple directions and are suitable for use in absorbent articles.

### BACKGROUND OF THE INVENTION

Outer covers for absorbent articles typically include a laminate comprising a film and a nonwoven. Absorbent articles sometimes fail to conform well to the wearer's body in response to body movements (e.g. sitting, standing, and walking). This conformity issue is further exacerbated by the fact that one type of absorbent article typically must fit many wearers of various shapes and sizes.

Stretch laminates with at least one incrementally stretched or SELFed nonwoven layer are described in WO 2017/004309 A1.

A need exists, therefore, for outer covers that exhibit low-force polydimensional stretch and recovery, and thus provide a conforming and discrete fit.

### SUMMARY OF THE INVENTION

The present invention meets the aforementioned need by providing elastomeric laminates comprising an elastomeric film and a suitable nonwoven, as described in claims 1 and 5. Particular embodiments are disclosed in the dependent claims. The film is primarily stretchable in the machine direction. Therefore, it would be expected that the film would limit the stretchability and recoverability of the laminate in other directions. Surprisingly, however, when laminated to a suitable nonwoven that is stretchable in the cross-direction, the resulting laminates are recoverably stretchable in essentially all directions. Therefore, the laminates are particularly suitable for use in absorbent articles where discrete fit and conformability are desired.

The present invention further describes methods of making the elastomeric laminates, wherein film is bonded to the nonwoven while stretched in the machine-direction, as defined in claim 8. Particular embodiments are defined in the dependent claims.

### BRIEF DESCRIPTION OF THE DRAWINGS

Figure 1 depicts one example of a laminate exhibiting biaxial stretching.
Figure 2 depicts one example of a laminate exhibiting polydimensional, or multiaxial, stretching.
Figure 3 is a graph depicting the cross-direction (CD) Load at 5% strain in N/cm (x-axis) as a function of machine-direction (MD) load at 5% strain in N/cm (y-axis) of laminates of the present invention (spunlace nonwovens) and comparative laminates (spunbond ("SB") and spunbond-meltblown-spunbond ("SMS") nonwovens).
Figure 4 shows the percentage of CD-elongation at peak strain (x-axis) as a function of the percentage of MD-elongation at peak strain (y-axis) of laminates of the present invention (spunlace nonwovens) and comparative laminates (SB and SMS nonwovens).
Figure 5 compares the CD extension in millimeters at 1000g (x-axis) as a function of the MD extension in millimeters at 1000g (y-axis) of laminates of the present invention (spunlace nonwovens) and comparative laminates (SB and SMS nonwovens).
Figure 6 depicts the percentage of applied stretch (x-axis) as a function of the MD extension in millimeters at 1000 g (y-axis) of laminates of the present invention (spunlace nonwovens) and comparative laminates (SB and SMS nonwovens).
Figure 7 depicts the CD/MD extension ratio (ER) at 1000g load of laminates of the present invention (spunlace nonwovens) and comparative laminates (SB and SMS nonwovens).
Figure 8 depicts the CD/MD elongation at peak ratio (EAPR) at peak strain of laminates of the present invention (spunlace nonwovens) and comparative laminates (SB and SMS nonwovens).

### DETAILED DESCRIPTION OF THE INVENTION

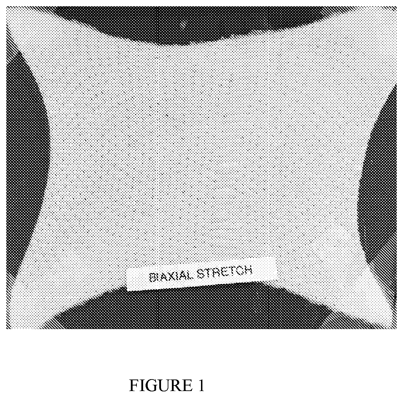

"Bi-axially stretchable," or variants thereof, means that the laminate is recoverably stretchable to at least twice its original length in two directions in the x-y plane, for example in the MD and in the CD, as depicted in Figure 1.

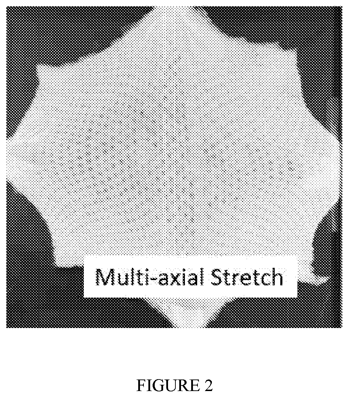

"Polydimensionally stretchable," "multi-axially stretchable," or variants thereof, means that the laminate is recoverably stretchable to at least twice its original length in at least three directions in the x-y plane, for example, as depicted in Figure 2.

"Uniformly polydimensionally stretchable" means that the laminate is recoverably stretchable after stretching to at least twice its original length in any direction.

"Recoverably stretchable," "recoverable," or variants thereof mean that when a laminate is stretched to at least twice its original length (200%), the laminate returns to no more than about 1.2 times the original length, as measured in the direction of the applied stretching force.

"Primarily stretchable," or variants thereof, mean that a film or a nonwoven substrate has a substantially greater degree of stretch in one particular direction (for example, in the CD or MD) than in other directions.

"Gsm" means grams per square meter, and is a measure of the basis weight, which is an industry standard term that quantifies the thickness or unit mass of a film or laminate product.

"Pre-activation," "activation," or variants thereof, mean a process by which the elastomeric film or material is rendered more easily stretchable prior to lamination, for example by stretching and allowing a film to relax. The films may be pre-activated in the CD and/or the MD.

The films of the present invention are elastomeric films, examples of which are disclosed in U.S. Patent Application 15/901,240, filed February 21, 2018.

The films may be multilayer or monolayer films and may comprise one or more styrenic block copolymers (SBCs) and/or olefinic block copolymers (OBCs). Suitable SBCs include but are not limited to styrene-butadiene-styrene (SBS), styrene-isoprene-styrene (SIS), styrene-isoprene-butylene-styrene (SIBS), styrene-ethylene-butylene-styrene (SEBS), styrene-ethylene-propylene (SEP), styrene-ethylene-propylene-styrene (SEPS), or styrene-ethylene-ethylene-propylene-styrene (SEEPS) block copolymer elastomers, and copolymers and mixtures of any of the foregoing. Although any SBC may be used, particularly useful SBCs in the films of the present invention are non-hydrogenated SBCs, including but not limited to SBS, SIS and SIBS. Non-limiting examples of SBCs suitable for use in the present invention include those available from Dexco Polymers, Plaquemine, Louisiana, for example, VECTOR 4111A and 7620.

Olefinic block copolymers (OBCs) suitable for use in one or more layers include polypropylene-based (also termed "propylene-rich") olefinic block copolymers such as those sold under the trade name INFUSE, nonlimiting examples of which include INFUSE 9507, 9100, 9507, 9107 and 5230, sold by The Dow Chemical Company of Midland, Michigan, and the trade names VISTAMAXX and IMPACT, for example VISTAMAXX 6102, available from ExxonMobil Chemical Company of Houston, Texas.

The total amount of SBCs and/or OBCs in the film or in an individual layer may be at least about 50%, from about 50% to about 100%, from about 60% to about 99%, from about 50% to about 95%, from about 55% to about 95%, from about 60% to about 95%, from about 65% to about 95%, from about 70% to about 95%, from about 75% to about 95%, from about 80% to about 95%, from about 70% to about 90%, or alternatively from about 80% to about 90%.

The outer layers (the A-layers, or skin layers) further each may comprise polypropylene in an amount of at least 10%, at least 15%, at least 20%, at least 25%, from about 1% to about 90%, from about 1% to about 85%, from about 1% to about 80%, or from about 1% to about 75%. **In** one embodiment, the polypropylene is present in an amount of at least 20%, and in another embodiment is present in an amount of from about 20% to about 85%.

The films have a basis weight that is both economical and suitable for use in absorbent articles. The films are primarily stretchable in one direction, which during manufacture, is typically the machine-direction. The films may have a basis weight of 100 gsm or less, 75 gsm or less, or 50 gsm or less, and alternatively from about 5 gsm to about 100 gsm, from about 15 gsm to about 75 gsm, from about 20 gsm to about 50 gsm, wherein all of the aforementioned ranges are inclusive of intermediate values and combinable. **In** one embodiment, the films have a basis weight of 50 gsm or less.

The present invention further includes laminates comprising the films described herein. The laminates comprise a substrate attached to one or both surfaces of the film, and may include laminates comprising more than one film and more than one substrate.

The substrate may be any woven or a nonwoven (NW) material that results in a laminate which is recoverably stretchable in multiple directions, including but not limited to spunbond (SB), meltblown (MB), or any combinations thereof (e.g., spunbond-meltblown-spunbond, or "SMS"), as well as spunlace, spinlace, airlaid, carded, and/or bicomponent nonwovens. Particularly suitable nonwovens include spunlace nonwovens such as those available from Suominen, Bethune, SC. In one embodiment, the substrate is primarily stretchable in one direction, which during manufacture of the laminates, is typically in the cross-direction. The substrate may have a basis weight of about 100 gsm or less, alternatively about 50 gsm or less, alternatively about 25 gsm or less, and alternatively from about 1 gsm to about 100 gsm, from about 25 gsm to about 75 gsm, and alternatively of from about 25 gsm to about 50 gsm, wherein all of the aforementioned ranges are inclusive of intermediate values and combinable. The substrate further may have a peak load of < 4 N/cm and/or a strain at peak of > 100%.

In one embodiment, the laminates of the present invention are substantially free of stranded elastomeric material.

In one embodiment, the laminates of the present invention are substantially free of adhesive.

In one embodiment, when laminated, the primary direction of stretch in the film is perpendicular to the primary direction of stretch of the nonwoven. The resulting laminate is biaxially and/or polydimensionally stretchable.

### Method of Making

One example of an apparatus suitable for making the films of the present invention is described in U.S. Patent 9,498,491 (Sablone et al.), available from Fameccanica Data SpA. Methods described generally therein also are suitable for producing the laminates of the present invention, with the exception of differences noted herein which contribute to the unique properties of the presently claimed laminates.

The films, of the present invention may be coextruded, and may be cast, blown, or formed by any other method which would result in the films described herein. In one embodiment, the films are pre-activated prior to lamination, for example by stretching in the machine-direction (MD), the cross-direction (CD), or both.

Prior to lamination, the film may be stretched in one direction. A nonwoven substrate, which is stretchable in a direction perpendicular to the direction in which the film is stretched, may be laminated to the film while the film is stretched. The nonwoven may be laminated while stretched or unstretched.

The substrate may be laminated to the film by a variety of means such as adhesive lamination, ultrasonic bonding, extrusion bonding, or other means that would be known to one of skill in the art. In one embodiment, the laminate is ultrasonically bonded, with the resulting laminates comprising ultrasonic welds, or bonds.

The films and/or laminates may be stretched in the cross-direction by using CD and/or MD intermeshing. The depth of intermeshing may vary from about 0.01 inches to about 0.250 inches, and in particular embodiments may be 0.120 inches, 0.140 inches, 0.160 inches or 0.180 inches (1 inch = 2.54 cm). Alternatively, the films and/or laminates may be stretched by means of diverging discs, as described for example in U.S. Patent Application 2018/0042778, Lenser et al., published February 15, 2018. In one embodiment, the diverging discs may be run at a slower speed than the anvil to provide simultaneous MD-stretch for the film during ultrasonic lamination.

The films and/or laminates of the present invention are useful for a variety of purposes, nonlimiting examples of which include use in articles such as personal hygiene products, including absorbent products. Non-limiting examples of absorbent products include diapers, training pants, adult incontinence pads and pants, swimwear, sanitary napkins, pantiliners, and/or absorbent pads or breathable shields to protect clothing from fluids, such as perspiration in specific areas of the body. The laminates may be used, for example, as backsheets, fasteners, waistbands, cuffs and/or ears. In one embodiment, the laminates are incorporated into an absorbent article such as a diaper or adult incontinence product.

### Examples

The laminates were made by stretching the film in the MD on a Fameccanica FMD-M2-00013 lamination system or other suitable production line and ultrasonically bonding the NW while the film is stretched. Three types of NW are used; 17 gsm SMS, 25 gsm SB (both available from Berry Global, Evansville IN), and 25 gsm spunlace (Suominen).

A three-layer film having an ABA layer construction is used. The film is an OBC-based elastic film. The skin layer 'A' is made of PE/PP blend and 1-10% of anti-block master batch and processing aid. The core layer 'B' is made of a propylene-based OBC blend comprising a mixture of INFUSE 9100, INFUSE 9507, INFUSE 9107, and/or DOW ELITE 5230. The core is about 85%-90% of the total thickness and remainder is the skin. Unless otherwise indicated, the basis weight of the films is between 35 gsm - 45 gsm.

The abovementioned films were not pre-activated. Pre-activating the film in the CD results in laminates with a higher stretch ratio in the CD and makes the polydimensional stretch more symmetric (data not shown).

A comparative film having a core comprising SBS and having the same skin, or A-layer, also was made (data not shown). However, the SBS film could not be stretched more than twice its original length before breaking and showed high frequency of thermal failure known as "pop-outs," wherein the film melts and tears causing areas in the laminate without film coverage. The sizes of the pop-outs typically exceeded 5 mm². In each combination of film/NW, the film was mechanically stretched in the MD at 300%, 400%, 500%, and 550% during lamination. The mechanical stretching was performed by running the anvil roll faster than the first nip roll. The resulting MD stretch of the laminate may be measured in three different ways:
1. The amount of stretch (estimated stretch %) when the final laminate stretched by hand is as follows: Two marks separated by 10 mm are marked on the laminate. The laminate is stretched by hand until reaching maximum stretch, i.e., the point at which the sample can no longer be stretched without incurring damage. The stretched distance is measured and divided by 10, and the result multiplied by 100 to obtain the stretch %.
2. The total extension (in mm) when the laminate is under 1000g tension.
3. The elongation (%) at peak strain in the tensile test.

The following defines relative directional stretchability: 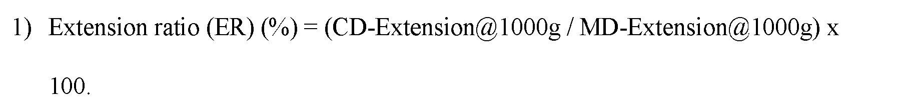 Extension ratio (ER) (%) = (CD-Extension@1000g / MD-Extension@1000g) x 100. 1) 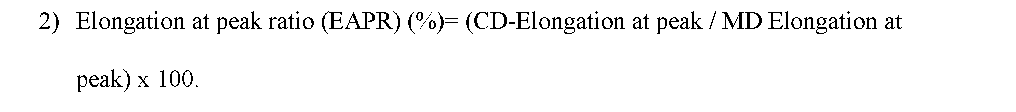 Elongation at peak ratio (EAPR) (%)= (CD-Elongation at peak / MD Elongation at peak) x 100. 2)

Table 1 summarizes the measured stretchability of the final product at different applied strains by the machine during lamination, where the applied stretch is derived from the ratio of the speed of the anvil roll to the speed of the nip roll. For the sake of clarity, a stretch of 50% means that the film is stretched by half its original length during lamination.

**Table 1: Stretchability of the final laminate measured by the first method.**

| Sample No. | NW | Applied Stretch during lamination | Measured Stretch by Hand |
|---|---|---|---|
| 1 | SMS-17 gsm | 300% | 100% |
| 2 | SMS-17 gsm | 400% | 150% |
| 3 | SMS-17 gsm | 500% | 170% |
| 4 | SMS-17 gsm | 550% | 170% |
| 5 | SB-25 gsm | 300% | 100% |
| 6 | SB-25 gsm | 400% | 150% |
| 7 | SB-25 gsm | 500% | 170% |
| 8 | Spunlace- 25 gsm | 300% | 100% |
| 9 | Spunlace- 25 gsm | 400% | 170% |
| 10 | Spunlace- 25 gsm | 500% | 100% |
| 11 | Spunlace- 25 gsm | 50% | 20% |

Table 2 summarizes the properties of the samples made on the FMD-M2-00013. The film was stretched in the machine direction (MD) then ultrasonically bonded while stretched. Thus, the final laminate is MD-stretchable as seen in Table 2. When a CD-stretchable spunlace NW is used, the final elastic laminate is stretchable in all directions (including CD and MD).

The CD-load at 5% strain should have a very low value if the product stretchable in the CD because both the film and the NW are easily deformed in the CD. On the other hand, if the film or the NW or both are not easily stretched in the CD, then the CD load at 5% strain is high and the laminate will only be stretchable in the MD. In this invention the film was stretched in the MD only before lamination by ultrasonic bonding. The data in the second column in Table 2 shows that CD loads at 5% strain of the samples made with the spunlace NW (samples 8 -11) have much lower values than CD-loads at 5% strain of the samples made with SB or SM nonwovens (samples 1-7). Samples 1-7 are stretchable in the MD direction only. All samples made by stretching the film in the MD before bonding and laminating. Therefore all samples have close low load values at 5% MD strain. In other words, the MD load at 5% appears to be deformed by the film only since the NW is corrugated and does not exert any significant force in the MD until the film is at least 100% stretched in the MD.

**Table 2: Sample Properties**

| Sample No. | CD-Load @ 5% (N/cm) | CD-Elong @ Peak (%) | MD-Load @ 5% (N/cm) | MD-Elong @ Peak (%) | CD-Ext @1000g (mm) | MD-Ext @1000g (mm) | CD/MD Ext. ratio |
|---|---|---|---|---|---|---|---|
| 1 | 2.18 | 82 | 0.23 | 199 | 2.0 | 22 | 9% |
| 2 | 2.91 | 74 | 0.22 | 190 | 2.0 | 27 | 7% |
| 3 | 2.92 | 76 | 0.20 | 198 | 2.2 | 30 | 7% |
| 4 | 2.25 | 75 | 0.20 | 178 | 2.2 | 29 | 8% |
| 5 | 2.30 | 73 | 0.27 | 184 | 2.1 | 22 | 10% |
| 6 | 2.33 | 75 | 0.19 | 186 | 2.0 | 27 | 8% |
| 7 | 2.62 | 73 | 0.21 | 165 | 2.1 | 27 | 8% |
| 8 | 0.64 | 226 | 0.26 | 202 | 19 | 32 | 59% |
| 9 | 0.55 | 241 | 0.25 | 200 | 25 | 35 | 72% |
| 10 | 0.25 | 219 | 0.34 | 332 | 19 | 50 | 38% |
| 11 | 0.25 | 217 | 0.50 | 89 | 27 | 21 | 128% |

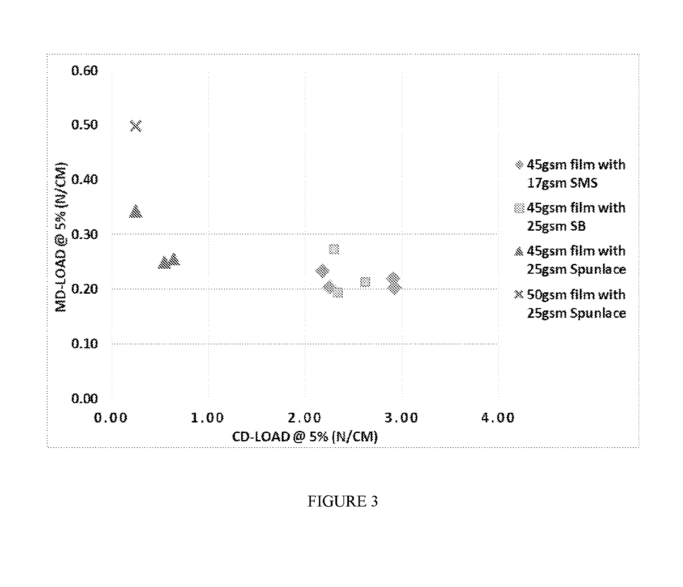

Figure 3 depicts the CD-Load and MD load of various samples. The samples made with spunlace NW have low 5% load values in both directions.

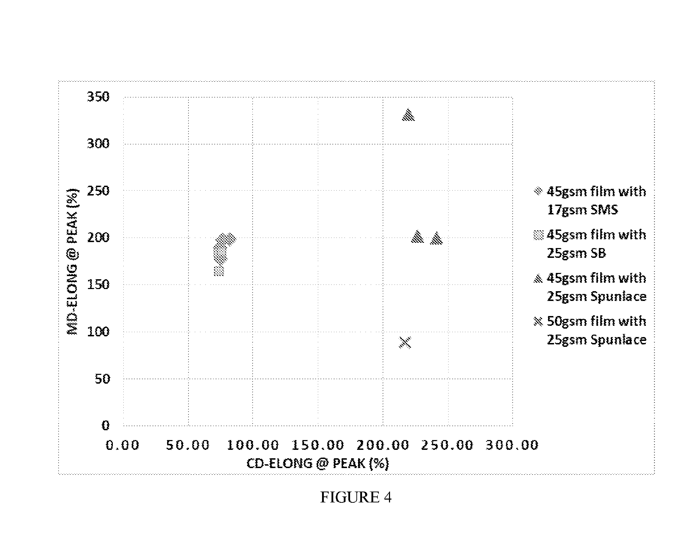

Figure 4 shows the elongation at peak map. Samples made with spunlace NW have high peak values in both directions.

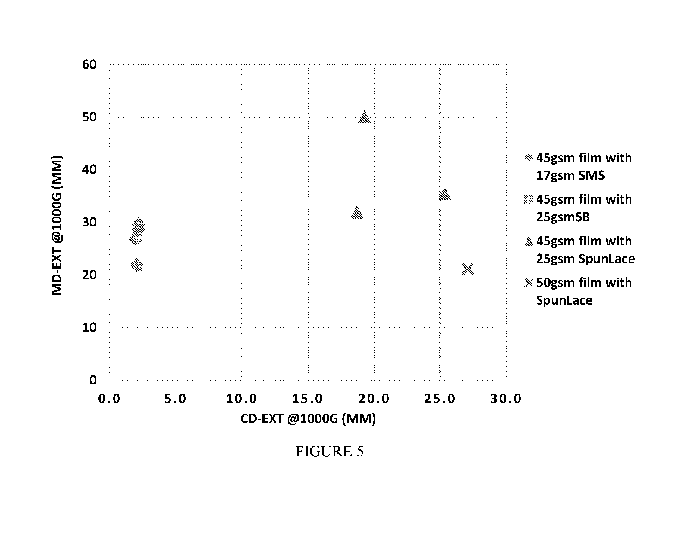

Figure 5 shows that laminate made with spunlace has high elongation at peak in the MD and CD. However, other samples have low values in the CD because they are not stretchable in the CD and high values in the MD because they are made to stretch in the MD.

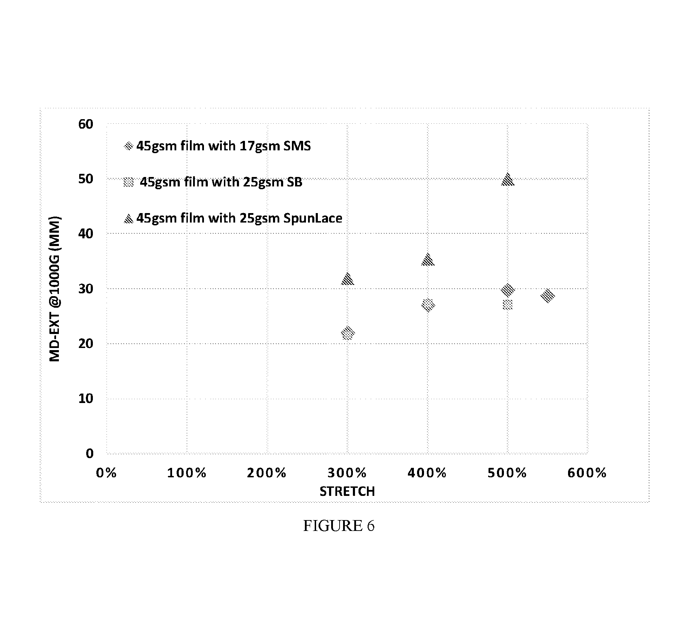

Figure 6 shows that the MD extension of the laminate is increased as the applied stretch is increased. There is no significant difference between the samples made with the SMS NW and the samples made with SB NW. However, the sample made with spunlace appears to have higher extension.

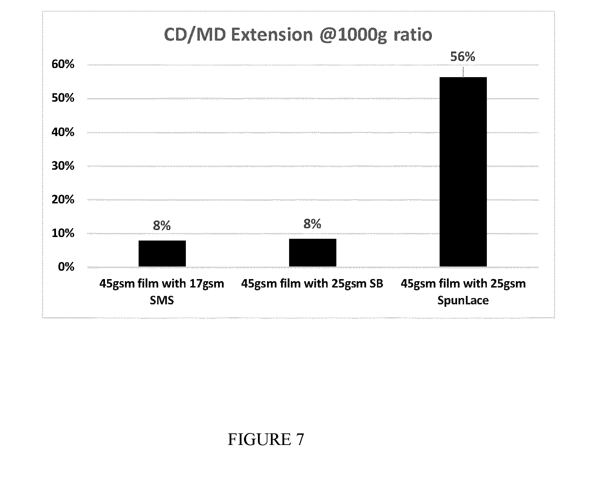

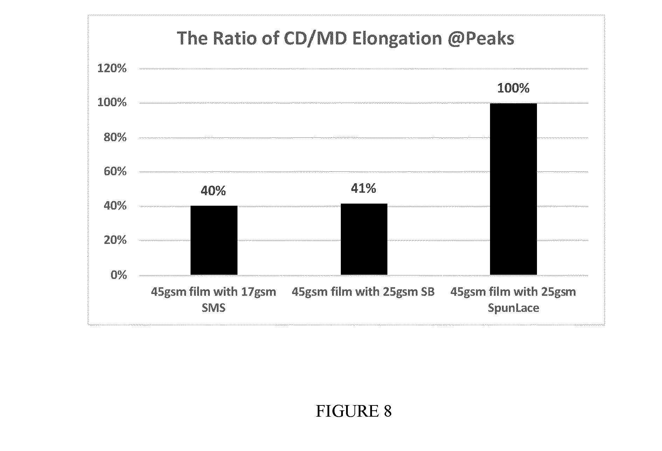

Figures 7 and 8 show that samples made with spunlace (CD-stretchable NW) have much higher values than the samples made with SMS NW or SB NW. In other words, laminates that are stretchable in both directions have high ER and EAPR.
All ranges are inclusive and combinable. To the extent a value is not explicitly listed, it is understood to be implied as an option if included in a recited range.

Whereas particular embodiments of the present invention have been illustrated and described, it would be obvious to those skilled in the art that various other changes and modifications can be made without departing from the scope of the invention. It is therefore intended to cover in the present claims all such changes and modifications that are within the scope of this invention.

## Claims

1. A laminate comprising at least one elastomeric film comprising an olefinic block copolymer, a styrenic block copolymer, or combinations thereof, which is primarily stretchable in a first direction; and at least one spunlace nonwoven substrate which is primarily stretchable in a second direction perpendicular to the first direction, wherein the laminate is polydimensionally stretchable;
wherein primary stretchable means that a film or a nonwoven substrate has a greater degree of stretch in one particular direction than in other directions, wherein polydimensionally stretchable means that the laminate is recoverably stretchable to at least twice its original length in at least three directions in the x-y plane.

2. The laminate of claim 1, wherein the ratio of cross-direction (CD) extension of the laminate to the machine-direction (MD) extension of the laminate is 30% or greater, wherein the cross-direction extension and the machine-direction extension are measured as described in the specification.

3. The laminate of claim 1, wherein the basis weight of the film is 50 gsm or less and wherein the basis weight of the nonwoven is 50 gsm or less.

4. The laminate of claim 1, wherein the laminate comprises ultrasonic welds.

5. A laminate comprising at least one elastomeric film comprising an olefinic block copolymer, a styrenic block copolymer or combinations thereof, which is primarily stretchable in a first direction, and at least one spunlace nonwoven substrate which is primarily stretchable in a second direction perpendicular to the first direction, wherein the laminate is stretchable in both the cross-direction and the machine direction, and wherein the ratio of cross-direction extension to the machine-direction extension is 30% or greater, wherein the cross-direction extension and the machine-direction extension are measured as described in the specification;
wherein primary stretchable means that a film or a nonwoven substrate has a greater degree of stretch on one particular direction than in other directions.

6. The laminate of claim 5, wherein the basis weight of the film is 50 gsm or less and the basis weight of the nonwoven is 50 gsm or less.

7. The laminate of claim 6, wherein the laminate comprises ultrasonic welds.

8. A method of making a laminate comprising the steps of:
a. Stretching an elastomeric film comprising an olefinic block copolymer, a styrenic block copolymer or combinations thereof in the machine direction; and,
b. Laminating the film to a spunlace nonwoven substrate which is primarily stretchable in the cross direction while the film is stretched in the machine-direction.

9. The method of claim 8, wherein the film is pre-activated; wherein pre-activation means a process by which the elastomeric film or material is rendered more easily stretchable prior to lamination.

10. The method of claim 8, wherein the film is not pre-activated; wherein pre-activation means a process by which the elastomeric film or material is rendered more easily stretchable prior to lamination.

11. The method of claim 8, wherein the film is laminated to the nonwoven by ultrasonic lamination, adhesive lamination, extrusion bonding, or combinations thereof.

12. The method of claim 8, wherein the film is stretched in the machine-direction by from 50% to 500%.

13. The method of claim 8, wherein the laminate is stretched in the cross-direction by intermeshing.

14. The method of claim 8, further comprising the step of incorporating the laminate into an absorbent article.

## Patentansprüche

1. Laminat, umfassend mindestens eine Elastomerfolie, die ein olefinisches Blockcopolymer, ein Styrol-Blockcopolymer oder Kombinationen davon umfasst und hauptsächlich in einer ersten Richtung dehnbar ist; und mindestens ein Spunlace-Vliesstoffsubstrat, das hauptsächlich in einer zweiten Richtung senkrecht zur ersten Richtung dehnbar ist, wobei das Laminat mehrdimensional dehnbar ist;
wobei hauptsächlich dehnbar bedeutet, dass eine Folie oder ein Vliesstoffsubstrat in einer bestimmten Richtung einen größeren Dehnungsgrad als in anderen Richtungen aufweist,
wobei mehrdimensional dehnbar bedeutet, dass das Laminat in mindestens drei Richtungen in der x-y-Ebene rückstellfähig auf mindestens das Zweifache seiner ursprünglichen Länge dehnbar ist.

2. Laminat nach Anspruch 1, wobei das Verhältnis der Dehnung des Laminats in Querrichtung (CD) zur Dehnung des Laminats in Maschinenrichtung (MD) 30 % oder mehr beträgt, wobei die Dehnung in Querrichtung und die Dehnung in Maschinenrichtung wie in der Beschreibung beschrieben gemessen werden.

3. Laminat nach Anspruch 1, wobei das Flächengewicht der Folie 50 g/m² oder weniger beträgt und wobei das Flächengewicht des Vliesstoffs 50 g/m² oder weniger beträgt.

4. Laminat nach Anspruch 1, wobei das Laminat Ultraschallschweißnähte umfasst.

5. Laminat, umfassend mindestens eine Elastomerfolie, die ein olefinisches Blockcopolymer, ein Styrol-Blockcopolymer oder Kombinationen davon umfasst und hauptsächlich in einer ersten Richtung dehnbar ist, und mindestens ein Spunlace-Vliesstoffsubstrat, das hauptsächlich in einer zweiten Richtung senkrecht zur ersten Richtung dehnbar ist, wobei das Laminat sowohl in Querrichtung als auch in Maschinenrichtung dehnbar ist und wobei das Verhältnis der Dehnung in Querrichtung zur Dehnung in Maschinenrichtung 30 % oder mehr beträgt, wobei die Dehnung in Querrichtung und die Dehnung in Maschinenrichtung wie in der Beschreibung beschrieben gemessen werden;
wobei hauptsächlich dehnbar bedeutet, dass eine Folie oder ein Vliesstoffsubstrat in einer bestimmten Richtung einen größeren Dehnungsgrad als in anderen Richtungen aufweist.

6. Laminat nach Anspruch 5, wobei das Flächengewicht der Folie 50 g/m² oder weniger beträgt und das Flächengewicht des Vliesstoffs 50 g/m² oder weniger beträgt.

7. Laminat nach Anspruch 6, wobei das Laminat Ultraschallschweißnähte umfasst.

8. Verfahren zur Herstellung eines Laminats, umfassend die Schritte:
a. Dehnen einer Elastomerfolie, die ein olefinisches Blockcopolymer, ein Styrol-Blockcopolymer oder Kombinationen davon umfasst, in Maschinenrichtung; und
b. Laminieren der Folie auf ein Spunlace-Vliesstoffsubstrat, das hauptsächlich in Querrichtung dehnbar ist, während die Folie in Maschinenrichtung gedehnt ist.

9. Verfahren nach Anspruch 8, wobei die Folie voraktiviert ist; wobei Voraktivierung einen Prozess bezeichnet, durch den die Elastomerfolie oder das Elastomermaterial vor dem Laminieren leichter dehnbar gemacht wird.

10. Verfahren nach Anspruch 8, wobei die Folie nicht voraktiviert ist; wobei Voraktivierung einen Prozess bezeichnet, durch den die Elastomerfolie oder das Elastomermaterial vor dem Laminieren leichter dehnbar gemacht wird.

11. Verfahren nach Anspruch 8, wobei die Folie durch Ultraschalllaminierung, Klebstofflaminierung, Extrusionsverbinden oder Kombinationen davon mit dem Vliesstoff laminiert wird.

12. Verfahren nach Anspruch 8, wobei die Folie in Maschinenrichtung um 50 % bis 500 % gedehnt wird.

13. Verfahren nach Anspruch 8, wobei das Laminat in Querrichtung durch Ineinandergreifen gedehnt wird.

14. Verfahren nach Anspruch 8, ferner umfassend den Schritt des Einbringens des Laminats in einen absorbierenden Artikel.

## Revendications

1. Stratifié comprenant au moins un film élastomère comprenant un copolymère bloc oléfinique, un copolymère bloc styrénique ou des combinaisons de ceux-ci, qui est principalement étirable dans une première direction ; et au moins un substrat non tissé spunlace qui est principalement étirable dans une deuxième direction perpendiculaire à la première direction, dans lequel le stratifié est étirable de manière polydimensionnelle ;
dans lequel principalement étirable signifie qu'un film ou un substrat non tissé présente un degré d'étirement plus élevé dans une direction particulière que dans d'autres directions,
dans lequel étirable de manière polydimensionnelle signifie que le stratifié est étirable avec récupération jusqu'à au moins deux fois sa longueur initiale dans au moins trois directions dans le plan x-y.

2. Stratifié selon la revendication 1, dans lequel le rapport de l'extension du stratifié dans le sens transversal (CD) à l'extension du stratifié dans le sens machine (MD) est supérieur ou égal à 30 %, dans lequel l'extension dans le sens transversal et l'extension dans le sens machine sont mesurées comme décrit dans la description.

3. Stratifié selon la revendication 1, dans lequel le grammage du film est inférieur ou égal à 50 g/m² et dans lequel le grammage du non-tissé est inférieur ou égal à 50 g/m².

4. Stratifié selon la revendication 1, dans lequel le stratifié comprend des soudures ultrasoniques.

5. Stratifié comprenant au moins un film élastomère comprenant un copolymère bloc oléfinique, un copolymère bloc styrénique ou des combinaisons de ceux-ci, qui est principalement étirable dans une première direction, et au moins un substrat non tissé spunlace qui est principalement étirable dans une deuxième direction perpendiculaire à la première direction, dans lequel le stratifié est étirable à la fois dans le sens transversal et dans le sens machine, et dans lequel le rapport de l'extension dans le sens transversal à l'extension dans le sens machine est supérieur ou égal à 30 %, dans lequel l'extension dans le sens transversal et l'extension dans le sens machine sont mesurées comme décrit dans la description ;
dans lequel principalement étirable signifie qu'un film ou un substrat non tissé présente un degré d'étirement plus élevé dans une direction particulière que dans d'autres directions.

6. Stratifié selon la revendication 5, dans lequel le grammage du film est inférieur ou égal à 50 g/m² et le grammage du non-tissé est inférieur ou égal à 50 g/m².

7. Stratifié selon la revendication 6, dans lequel le stratifié comprend des soudures ultrasoniques.

8. Procédé de fabrication d'un stratifié comprenant les étapes consistant à :
a. étirer un film élastomère comprenant un copolymère bloc oléfinique, un copolymère bloc styrénique ou des combinaisons de ceux-ci dans le sens machine ; et
b. stratifier le film sur un substrat non tissé spunlace qui est principalement étirable dans le sens transversal tandis que le film est étiré dans le sens machine.

9. Procédé selon la revendication 8, dans lequel le film est préactivé ; dans lequel préactivation signifie un processus par lequel le film élastomère ou le matériau élastomère est rendu plus facilement étirable avant la stratification.

10. Procédé selon la revendication 8, dans lequel le film n'est pas préactivé ; dans lequel préactivation signifie un processus par lequel le film élastomère ou le matériau élastomère est rendu plus facilement étirable avant la stratification.

11. Procédé selon la revendication 8, dans lequel le film est stratifié sur le non-tissé par stratification ultrasonique, stratification adhésive, liaison par extrusion ou des combinaisons de celles-ci.

12. Procédé selon la revendication 8, dans lequel le film est étiré dans le sens machine de 50 % à 500 %.

13. Procédé selon la revendication 8, dans lequel le stratifié est étiré dans le sens transversal par engrènement.

14. Procédé selon la revendication 8, comprenant en outre l'étape d'incorporation du stratifié dans un article absorbant.
